# EUROPÄISCHE PATENTSCHRIFT

(11) **EP 1 475 083 B1**
(45) Veröffentlichungstag und Bekanntmachung des Hinweises auf die Patenterteilung: **26.12.2007**
(21) Anmeldenummer: 04010510.8
(22) Anmeldetag: 11.02.2002
(51) Int. Cl.: A61K 9/107, A61K 31/122

(54) **Verfahren zur Herstellung eines Wirkstoffkonzentrats sowie Wirkstoffkonzentrat**
Method for producing an active ingredient concentrate, and active ingredient concentrate
Procédé de production d'un concentré de principe actif et concentré de principe actif

(30) Priorität: 11.02.2001 DE 10109708; 22.02.2001 DE 10108614
(43) Veröffentlichungstag der Anmeldung: 10.11.2004
(62) Teilanmeldung aus: 02719799.5
(73) Patentinhaber: Aquanova AG, 64295 Darmstadt (DE)
(72) Erfinder: Behnam, Dariush, 64380 Rossdorf (DE)
(74) Vertreter: Blumbach - Zinngrebe

(56) Entgegenhaltungen:
- WO-A-01/28520
- WO-A-99/57995

## Beschreibung

Die Erfindung betrifft ein wasserlösliches Konzentrat, welches Ubichinon Q₁₀ der in Wasser unlöslich oder nur schwer löslich ist, sowie einen Lösungsvermittler aufweist, sowie Verfahren zur Herstellung der Konzentrate.

Fettlösliche Verbindungen wie Coenzym Q₁₀ wird in Abhängigkeit vom Vorhandensein von Gallensalzen und Enzymen der Bauchspeicheldrüse absorbiert. Dem Absorptionsprozess geht ein Vorgang der sogenannten Micellenbildung im Darm voraus, der erforderlich ist, damit die fettlöslichen Verbindungen "verpackt" werden und auf diese Weise verschiedene Barrieren der Darmmucosa überwinden können.

Ist die Sekretion von Gallenflüssigkeit oder Enzymen der Bauchspeicheldrüse gestört, so resultiert daraus eine sogenannte Maldigestion oder Malabsorption fettlöslicher Verbindungen. Das beste Beispiel hierfür ist der Krankheitszustand der Zystischen Fibrose, bei der aufgrund mangelnder Bereitstellung von Bauchspeicheldrüsenenzymen die Resorption fettlöslicher Verbindungen nur noch in sehr geringem Umfang möglich ist.

Die Besonderheiten der Absorption fettlöslicher Mikronährstoffe zeigt sich auch daran, dass die Aufnahme immer dann steigt, wenn gleichzeitig Fett angeboten wird. Fett begünstigt einerseits die Abgabe von Gallensäure und Enzymen der Bauspeicheldrüse und andererseits die Bildung von Micellen, die dann die besagten fettlöslichen Mikronährstoffe enthalten.

Nachdem die fettlöslichen Verbindungen von den Darmzellen aufgenommen worden sind, liegen sie dort in freier Form vor, d.h. sie sind nicht mehr an micellare Bestandteile gebunden. In dieser freien Form werden sie dann erneut "wasserlöslich" gemacht, indem sie in innerhalb der Darmzellen gebildete Transporter eingebaut (Lipoproteine - Chylomikronen) und dann über die großen Lymphwege ins Blut abgegeben werden.

Um lipophile Verbindungen aufnehmen zu können, muss sie der Organismus also in zwei Schritten wasserlöslich machen. Der erste Schritt erfolgt im Darm durch die Bildung der Micellen, aus denen dann die Substanz in der Darmzelle wieder freigesetzt wird, und der zweite Schritt ist die Bildung von Lipoproteinen zum Transport im Blut. Daher werden lipophile Substanzen, die wasserlöslich gemacht worden sind (klare Lösungen), nicht aber solche, die im wässrigen Medium lediglich dispergiert sind (trübe Lösungen), vom Organismus rascher und effizienter absorbiert als die ursprünglich lipophile Substanz.

Über die Bioverfügbarkeit wasserlöslich gemachter lipophiler Mikronährstoffe (klare Lösungen) liegen nur wenig Daten vor. Ein Verfahren zur Prüfung der Bioverfügbarkeit solcher Verbindungen sind sogenannte in-vitro-Dissolutionsverfahren. Hierbei wird festgestellt, inwieweit sich eine Verbindung im wässrigen Kompartiment löst bzw. inwieweit sie aus einer bestimmten galenischen Zubereitungsform freigesetzt wird. Aus US-6,048,566 ist bekannt, dass wasserlöslich gemachtes Q₁₀ im Gegensatz zu Q₁₀ aus öligen Lösungen oder Dispersionen (trübe Lösungen) zu 100 Prozent freigesetzt wird. Dies bedeutet aber, dass das so applizierte Q₁₀ bereits in höherer Konzentration in freier Form im Darmlumen vorliegt und auf diese Weise nicht erst durch Micellenbildung oder durch Abbau der das Q₁₀ umgebenden Lipide freigesetzt werden muss.

WO-A-9957995 offenbart ein Verfahren zur Herstellung einer klaren Flüssigkeit, dadurch gekennzeichnet, dass das Coenzym Q10 unter Erwärmen zu einer Mischung von Miglyol 812, Polysorbat 80 sowie in Ethanol gelöstem Lecithin hinzugegeben wird.

Der Erfindung liegt deshalb die Aufgabe zugrunde, die Bioverfügbarkeit von ursprünglich in Wasser nicht oder nur schwer löslichen, physiologisch bedeutsamen Substanzen Ubichinonen (z.B. Coenzym Q₁₀) zu verbessern und die industrielle Verarbeitung dieser Substanzen technologisch zu erleichtern.

Der Erfindung liegt auch die Aufgabe zugrunde, bei der Herstellung der Konzentrate die kleinstmögliche Menge an Lösungsvermittlern also insbesondere Polysorbaten - unter Berücksichtigung eines Toleranzbereichs zur Sicherung der vollständigen, stabilen Wasserlöslichkeit - einzusetzen, so dass die ADI-Werte (ADI=Acceptable Daily Intake) für die Polysorbate gemäß JECFA (=Joint FAO/WHO Expert Committee on Food Additives) und SCG-Substanzen (SCG = Scientific Committee on Food (EU)) wie Ubichinonen (z.B. Coenzym Q₁₀) deutlich unterschritten werden.

Zur Erfüllung dieser Aufgaben ist erfindungsgemäß vorgesehen, dass der inWasser nicht oder nur schwer lösliche Wirkstoff in einen Überschuß an erwärmtem körperverträglichen Lösungsvermittler mit einem HLB-Wert zwischen 6 und 19, insbesondere Polysorbat 20 oder Polysorbat 80, eingetragen, die Mischung solange in der Wärme gerührt wird, bis sich ein klares zähflüssiges Zwischenprodukt ergibt, und das Zwischenprodukt anschließend auf Zimmertemperatur abgekühlt wird. Soll der Wirkstoff in wässriger Phase bereitgestellt werden, wird dem warmen Zwischenprodukt warmes destilliertes Wasser in solcher Menge beigegeben, wie der erwünschten Wirkstoffkonzentration entspricht, bis zur Homogenität gerührt und die so entstandene Phase rasch auf Zimmertemperatur abgekühlt.

Soll die Phase wasserfrei sein, wird dem warmen Zwischenprodukt ein warmes Triglycerid, beispielsweise ein leichtes Pflanzenöl mit hohem Linolsäuregehalt sowie warmes Polysorbat in solcher Menge zugegeben, daß sich die gewünschte Wirkstoffkonzentration ergibt, und erneut in der Wärme bis zur Klarheit des Konzentrats gerührt. Nach Abkühlen liegt der Wirkstoff in wasserfreier, jedoch in Wasser beliebig löslicher Phase vor. Diese eignet sich besonders zur Darreichung des Wirkstoffes in Kapselform, welche nur einen sehr geringen Wassergehalt auf Dauer toleriert.

Sowohl die wasserfreie wie die wässrige Phase sind in Wasser bzw. Fett und/oder Öl löslich.

Wie Messungen mittels einphasiger Chromatographie zeigen, liegt der Wirkstoff sowohl in der wässrigen wie in der wasserfreien Phase in von Polysorbat eingehüllten Molekülaggregaten vor, wobei die Polysorbathülle jeweils einen Durchmesserbereich von etwa 30 nm aufweist, die Polysorbathülle mit eingeschlossenem Molekülaggregat also als eine Micelle anzusehen ist.

Im physiologischen Bereich hat die erfindungsgemäße Micellenbildung eine wesentlich verbesserte Bioverfügbarkeit des Wirkstoffes zur Folge. Der in Wasser nicht oder nur schwer lösliche Wirkstoff braucht nicht erst durch Einwirkung von Gallensekreten für das Darmlumen aufnahmefähig gemacht zu werden.

Ausrührungsbeispiele der Erfindung sind in den Unteransprüchen angegeben. In der beigefügten Zeichnung zeigen
Figur 1 eine Darstellung gemessener Radiusverteilungen von Micellen;
Figur 2 eine schematische Erläuterung von Wirkstoffmicellen am Beispiel des Coenzyms Q₁₀ und
Figur 3 eine schematische Erläuterung der Anordnung von Micellen, die einen Wirkstoff (Coenzym Q₁₀) sowie einen Hilfsstoff(Linolsäure) enthalten;

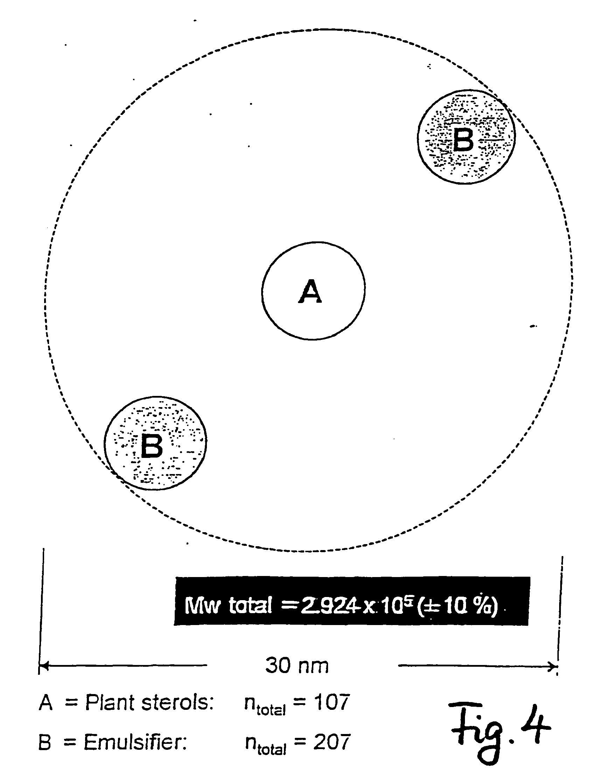

Figur 4: eine schematische Darstellung von Phytosterinmicellen;

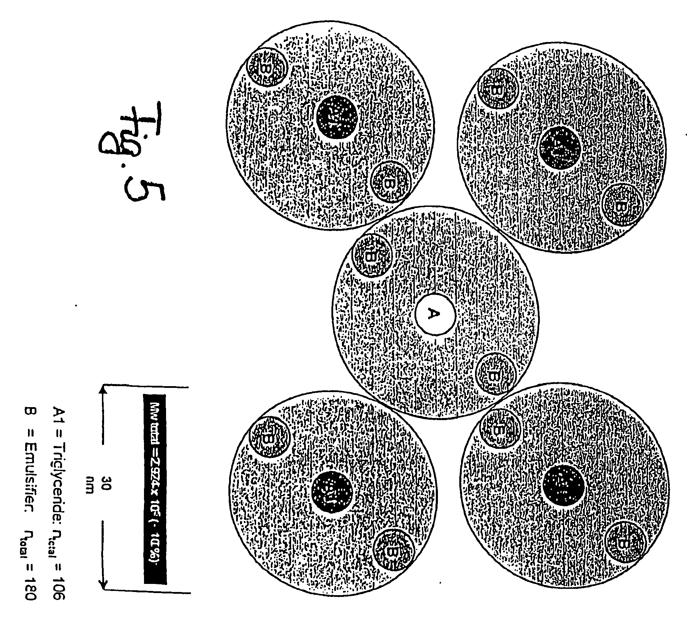

Figur 5: eine schematische Darstellung der Verteilung zusätzlicher Linolsäuremicellen um eine Phytosterinmicelle, und

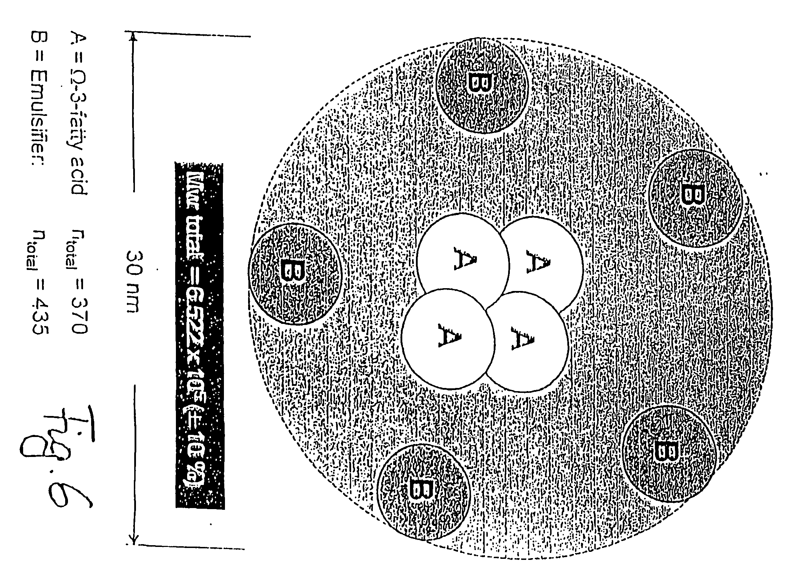

Figur 6: eine schematische Darstellung einer Micelle einer ω 3 Fettsäure.

### Beispiel 1: Coenzym Q₁₀

In 77 Massenteile von auf 85°C erwärmtes Polysorbat 80 werden 23 Massenteile Coenzym Q₁₀ eingetragen und die Mischung wird etwa 5 min bei 85°C gerührt, bis sich eine klare zähflüssige Masse leicht gelblicher Farbe ergibt Die Massenanteile im Zwischenprodukt von Coenzym Q₁₀ zu Polysorbat 80 betragen 1 : 3,35 und das Verhältnis der Molekülzahlen beträgt 1 : 2,56, da das Molekulargewicht von Coenzym Q₁₀ 863,36 und dasjenige von Polysorbar 80 1130,00 beträgt.

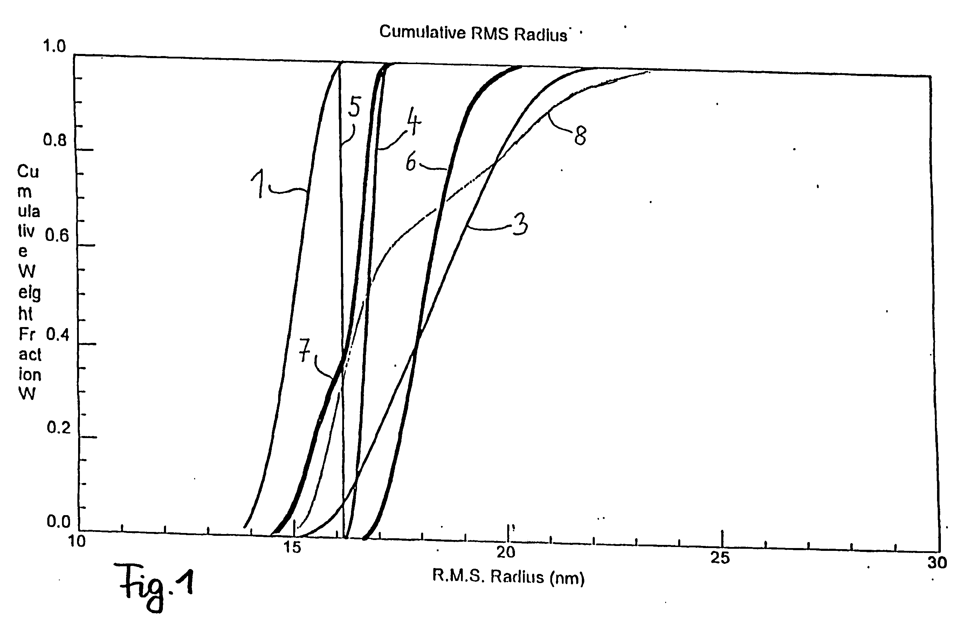

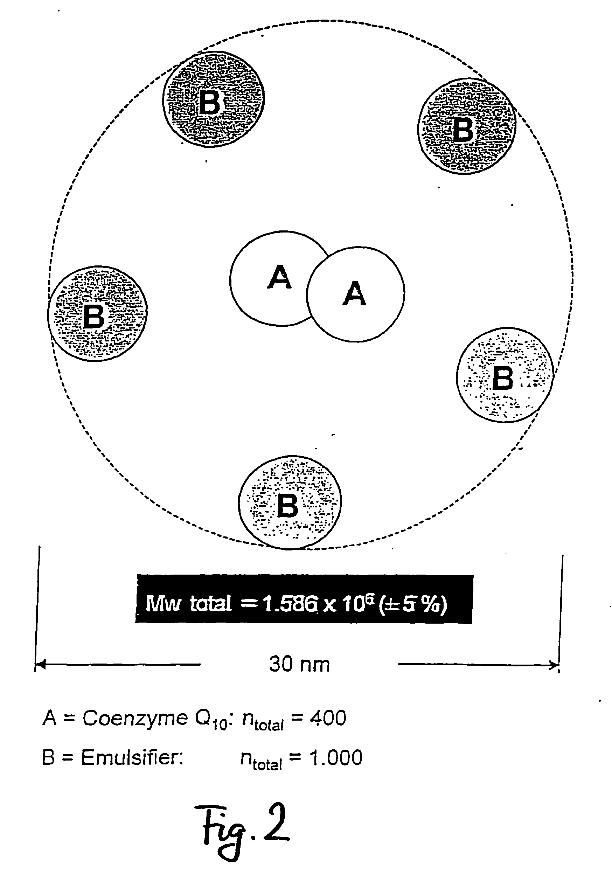

Zur Gewinnung einer wässrigen Phase von Q₁₀ aus dem Zwischenprodukt, welche eine Q₁₀ Konzentration von etwa 3% enthält, wird dem warmen Zwischenprodukt etwa 865 Gewichtsteile an warmem Wasser zugesetzt und in der Wärme erneut gerührt, bis sich eine klare Flüssigkeit ergibt. Anschließend wird die Flüssigkeit rasch (beispielsweise innerhalb etwa 1 bis 2 Minuten) auf Zimmertemperatur (etwa 20°C) zur fertigen wässrigen Phase des Coenzyms Q₁₀ heruntergekühlt. Der durchschnittliche in der Phase vorliegende Partikelradius wurde durch Feld-Fluß-Fraktionierung (FFF) mit an die Chromatographiesäule angekoppeltem DAWN EOS-Detektor der Firma Wyatt Technologie Deutschland GmbH erfaßt. Wie die Kurve 1 in Figur 1 zeigt, liegt der Radius in Abhängigkeit von der kumulativen Gewichtsfraktion zwischen etwa 14 nm und etwa 16 nm und damit im Größenbereich der Micellen. Aus dem hierbei bestimmten durchschnittlichen Micellengewicht von etwa 1,586 x 10⁶ Einheiten läßt sich errechnen, daß jede Micelle im Kern zwei Molekülaggregate von insgesamt etwa 400 Molekülen Coenzym Q₁₀ aufweist, welcher von fünf untereinander gleichartigen Molekülaggregaten Polysorbat 80 von insgesamt etwa 1000 Molekülen Polysorbat 80 umgeben ist, wie schematisch in Figur 2 dargestellt ist.

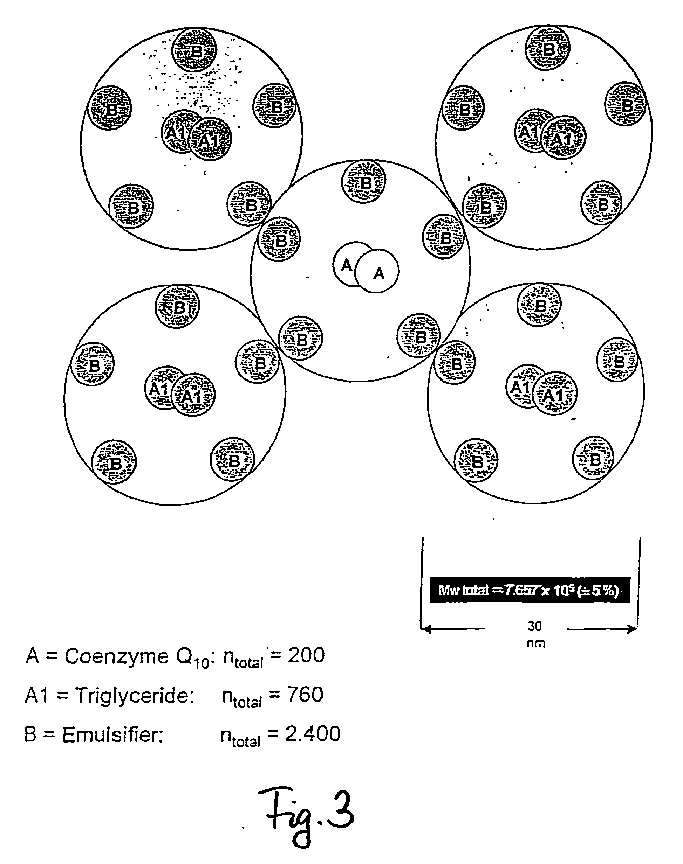

Zur Gewinnung der wasserfreien Phase von 5 Gew% Coenzym Q₁₀ werden dem warmen Zwischenprodukt soviel Triglycerid und Polysorbat 80 in der Wärme (85°C) zugesetzt, daß die Mischung etwa 5 Teile Coenzym Q₁₀, etwa 16 Teile Triglycerid und etwa 79 Teile Polysorbat 80 aufweist. Als Triglycerid wird hier Distelöl eingesetzt, welches nach den Leitsätzen für Speisefette und Speiseöle vom 29/30.11.1983 in der am 2/3.12.1986 geänderten Fassung (GMB1. Nr. 21, Seite 379 vom 1.8.1987) einen hohen Linolsäuregehalt, nämlich von etwa 67,8% bis etwa 83,2%, aufweist. Linolsäure empfiehlt sich wegen seiner dem Coenzym Q₁₀ ähnlichen Molekülgröße (Molekulargewicht der Linolsäure 725). Die Mischung wird bis zur Klarheit in der Wärme gerührt und alsdann langsam abgekühlt Man erhält eine wasserfreie Phase von Coenzym Q₁₀, deren Partikelgröße aufgrund der genannten Messungen ebenfalls im Micellenbereich liegt. Im Unterschied zur wässrigen Phase wird mit der genannten Methode ein Micellendurchmesseer von durchschnittlich 7,657 x 10⁵ gemessen aus welchem sich ergibt, daß jede Micelle im Kern etwa 200 Coenzym Q₁₀ Moleküle und fünf umlagernde Molekülaggregate von insgesamt etwa 480 Molekülen Polysorbat 80 aufweist, wobei diese Micelle von vier weiteren untereinander gleichartigen Micellen umgeben ist, von denen jede im Kern etwa 190 Moleküle Distelöl bezw. Linolsäure und eine Polysorbathülle aus fünf Molukülaggregaten von insgesamt etwa 480 Molekülen Polysorbat aufweist Eine schematische Darstellung dieser Micellenausbildung zeigt Figur 3.

Die wasserfreie Phase ist ausgezeichnet lagerfähig und läßt sich in Wasser bei Körpertemperatur beliebig lösen Sie ist deshalb als Zusatz zu Nahrungsergänzungsmitteln geeignet, die üblicherweise in Gelatinekapseln angeboten werden. Eine Erklärung für die besondere Beständigkeit der wasserfreien Phase kann unter Umständen darin gesehen werden, daß die zentrale, das Coenzym Q₁₀ enthaltende Micelle durch die vier umgebenden, den Hilfsstoff Distelöl d.h. im wesentlichen also Linolsäure enthaltenden Micellen vor dem Eindringen insbesondere polarer Moleküle wie H₂O weitgehend geschützt ist

Die erfindungsgemäß gebildeten Micellen sind chemisch, mikrobiologisch, mechanisch und thermisch sehr stabil. Sie enthalten anteilig eine viel größere Menge an meist lipophilen Wirkstoffen als vergleichbare Liposomen. Die erfindungsgemäßen Wirkstoffkonzentrate bzw. die gebildeten Wirkstoffphasen lassen sich mit Vorteil Lebensmitteln, Nahrungergänzungsmitteln, Haut-, Haar- und Zahnpflegemitteln sowie kosmetischen oder pharmazeutischen Mitteln zusetzen. Die Wirkstoftkonzentrate sind absolut magensäurestabil. Durch die Micellenbildung sind die Wirkstoffe für den Organismus wesentlich schneller verfügbar als in Emulsionen verabreichte Wirkstoffe. Die Resorption im intestinalen Bereich macht eine Beteiligung der Gallensäure überflüssig.

## Patentansprüche

1. Wasserlösliches Konzentrat des Ubichinon Q₁₀ welches aus einer Lösung von Q₁₀ in erwärmtem Polysorbat, und einem Gehalt eines Hilfsstoffes in Form eines Triglycerids besteht.

2. Konzentrat nach Anspruch 1, bestehend aus etwa 5 Gewichtsteilen Q₁₀, etwa 16 Gewichtsteilen Triglycerid und etwa 79 Gewichtsteilen Polysorbat 80.

3. Konzentrat nach Anspruch 1 oder 2, bei dem das Triglycerid Distelöl ist.

4. Verfahren zur Herstellung eines Konzentrats nach einem der vorstehenden Ansprüche, **dadurch gekennzeichnet, dass** der Mischung aus Q₁₀ und Polysorbat 80 in der Wärme ein Hilfsstoff in Form eines Triglycerids zugesetzt und bis zur Klarheit gerührt wird.

5. Verfahren nach Anspruch 4 mit Distelöl als Triglycerid.

6. Verfahren nach Anspruch 4 oder 5, bei dem das Polysorbat auf etwa 80 °C bis etwa 100 °C erwärmt wird.

7. Verwendung des Konzentrats nach einem der Ansprüche 1-3 als nicht therapeutischer Zusatz zu Nahrungsergänzungsmitteln in Kapselform.

## Claims

1. Water-soluble concentrate of ubiquinone Q₁₀, which consists of a solution of Q₁₀ in heated polysorbate and a content of an excipient in the form of a triglyceride.

2. Concentrate according to Claim 1, consisting of about 5 parts by weight of Q₁₀, about 16 parts by weight of triglyceride and about 79 parts by weight of polysorbate 80.

3. Concentrate according to Claim 1 or 2, in which the triglyceride is thistle oil.

4. Method for producing a concentrate according to any of the above claims, **characterized in that** an excipient in the form of a triglyceride is added to the mixture of Q₁₀ and polysorbate 80 at elevated temperature and the mixture is stirred until it is clear.

5. Method according to Claim 4 with thistle oil as the triglyceride.

6. Method according to Claim 4 or 5, in which the polysorbate is heated to about 80°C to about 100°C.

7. Use of the concentrate according to any of Claims 1-3 as a non-therapeutic additive to food supplements in capsule form.

## Revendications

1. Concentré hydrosoluble d'ubiquinone Q₁₀, qui est constitué d'une solution de Q₁₀ dans du Polysorbate chauffé et d'une teneur en une substance auxiliaire sous forme d'un triglycéride.

2. Concentré suivant la revendication 1, constitué d'environ 5 parties en poids de Q₁₀, d'environ 16 parties en poids de triglycéride et d'environ 79 parties en poids de Polysorbate 80.

3. Concentré suivant la revendication 1 ou 2, dans lequel le triglycéride est de l'huile de chardon.

4. Procédé de production d'un concentré suivant l'une des revendications précédentes, **caractérisé en ce que** le mélange de Q₁₀ et de Polysorbate 80 est additionné à chaud d'une substance auxiliaire sous forme d'un triglycéride et agité jusqu'à ce qu'il soit limpide.

5. Procédé suivant la revendication 4, utilisant l'huile de chardon comme triglycéride.

6. Procédé suivant la revendication 4 ou 5, dans lequel le Polysorbate est chauffé à une température d'environ 80°C à environ 100°C.

7. Utilisation du concentré suivant l'une des revendications 1 à 3 comme additif non thérapeutique pour compléments nutritionnels sous forme de gélules.
